(19) Europäisches Patentamt
European Patent Office
Office européen des brevets

(11) **EP 3 070 449 A1**

(12) **EUROPEAN PATENT APPLICATION**

(43) Date of publication:
21.09.2016 Bulletin 2016/38

(51) Int Cl.:
*G01L 11/02* (2006.01)      *G01H 9/00* (2006.01)
*G01L 1/24* (2006.01)

(21) Application number: 16160451.7

(22) Date of filing: 15.03.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 16.03.2015 US 201562133809 P

(71) Applicant: **Total Wire Corporation**
**Richardson, TX 75080 (US)**

(72) Inventor: **Zhou, Gan**
**Plano, TX 75074 (US)**

(74) Representative: **Swindell & Pearson Limited**
**48 Friar Gate**
**Derby DE1 1GY (GB)**

(54) **REMOTE PRESSURE SENSOR AND METHOD OF OPERATION THEREOF**

(57)     Pressure sensors, a method of sensing pressure and a method of determining a change in birefringence of a polarization maintaining (PM) optical fiber. In one embodiment, the pressure sensor includes: (1) a source of laser light, (2) a polarization module coupled to the source and configured to modulate a polarization state of the light, (3) a PM optical fiber configured to receive the light into a proximal end thereof and having a sensor coupled to a distal tip of the PM optical fiber and having a pressure-dependent optical anisotropy and (4) a detector configured to receive the light back from the sensor via the proximal end and provide a signal based thereon that indicates a pressure on the sensor.

Fig. 1

EP 3 070 449 A1

## Description

## TECHNICAL FIELD

This application is directed, in general, to pressure sensing and, more specifically, to a remote pressure sensor and a method of operating the same to sense pressure remotely.

## BACKGROUND

Remote sensing of pressure is an important function with applications in a wide range of fields, from the oil and gas industry to medical procedures and healthcare. In coronary artery disease treatment, for example, it is often necessary to measure the intravascular blood pressure profile along a diseased vessel region that has significant plaque buildup. Such information about intravascular pressure, and the "fractional flow reserve" derived from it, are important indicators that help guide a clinician's decision on whether certain therapeutic measures, such as stenting, should be taken.

Some conventional pressure sensors are based on piezoelectric material. Piezoelectric sensors are accurate, but they are sensitive to environmental factors, such as temperature change, local stress, and electromagnetic interference. In addition, their construction essentially precludes adapting them to perform additional functions, such as ultrasonic imaging.

Other conventional pressure sensors employ an optical fibers, with a Fabry-Perot etalon coupled to the tip of the fiber. Laser interferometry is used to detect a change in Fabry-Perot cavity length caused by a change in pressure. Unfortunately, optical fiber sensors require a microscopic-scale air cavity which is difficult to fabricate. In addition, detecting the cavity length is complicated, requiring white-light interferometry, and the performance of optical fiber sensors has yet to match the accuracy of the piezoelectric sensors.

## SUMMARY

One aspect provides a pressure sensor, a method of sensing pressure and a method of determining a change in birefringence of a polarization maintaining (PM) optical fiber. In one embodiment, the pressure sensor includes: (1) a source of laser light, (2) a polarization module coupled to the source and configured to modulate a polarization state of the light, (3) a PM optical fiber configured to receive the light into a proximal end thereof and having a sensor coupled to a distal tip of the PM optical fiber and having a pressure-dependent optical anisotropy and (4) a detector configured to receive the light back from the sensor via the proximal end and provide a signal based thereon that indicates a pressure on the sensor.

Another embodiment of the pressure sensor includes: (1) a source of laser light of first and second wavelengths, (2) a polarization module coupled to the source and configured to modulate a polarization state of the light, (3) a PM optical fiber configured to receive the light into a proximal end thereof and having a sensor coupled to a distal tip of the PM optical fiber and having a pressure-dependent optical anisotropy, (4) a wavelength-selective coating associated with the sensor and configured substantially to prevent the light of the second wavelength from entering the sensor and (5) a detector configured to: (5a) receive the light of the first wavelength back from the sensor via the proximal end and provide a signal based thereon that indicates the pressure and (5b) receive the light of the second wavelength back from the distal tip via the proximal end and provide a signal based thereon that indicates the birefringence of the PM optical fiber.

Another aspect provides a method of sensing pressure. In one embodiment, the method includes: (1) generating laser light, (2) modulating a polarization state of the light, (3) receiving the light into a proximal end of a PM optical fiber having a sensor coupled to a distal tip thereof, the sensor having a pressure-dependent optical anisotropy, (4) receiving the light back from the sensor via the proximal end and (5) providing a signal based thereon that indicates a pressure on the sensor.

Still another aspect provides a method of reducing an influence of birefringence in a PM optical fiber on a pressure measurement obtained therethrough. In one embodiment, the method includes: (1) generating laser light of first and second wavelengths, (2) modulating a polarization state of the light, (3) receiving the light into a proximal end of the PM optical fiber having an optical sensor coupled to a distal tip thereof, the sensor having a pressure-dependent optical anisotropy, (4) substantially preventing the light of the second wavelength from entering the sensor, (5) receiving the light of the first wavelength back from the sensor via the proximal end, (6) receiving the light of the second wavelength back from the distal tip via the proximal end and (7) providing a signal based on the light that indicates a pressure at the sensor that is substantially independent of a change in a birefringence of the PM optical fiber.

## BRIEF DESCRIPTION

Aspects of the disclosure can be better understood with reference to the following drawings. The components in the drawings are not necessarily to scale, emphasis instead being placed upon clearly illustrating the principles of the present disclosure. Moreover, in the drawings, like reference numerals designate corresponding parts throughout the several views, and in which:

Fig. 1 is a schematic block diagram of one embodiment of a pressure sensor;
Fig. 2 is a diagram of certain parts of one embodiment of a probe of the pressure sensor of Fig. 1;

Fig. 3 is a diagram of one embodiment of a distal tip of the probe of Fig. 2;

Fig. 4 is a diagram showing the details of one embodiment of the construction of the probe of Fig. 2;

Figs. 5A and 5B are cross-sectional views of the sensor of Fig. 4, taken along respective lines 5A and 5B thereof;

Fig. 5C is a cross-sectional view of the sensor of Fig. 4, taken along lines 5B and showing an alternative embodiment of the view of Fig. 5B;

Fig. 6 is a diagram of one embodiment of a polarization module;

Figs. 7A-7D are diagrams illustrating several examples of simulated results of pressure measurements;

Fig. 8 is a diagram of one embodiment of a pressure probe that can be calibrated using a second wavelength; and

Fig. 9 is a flow diagram of one embodiment of a method of operating a remote pressure sensor to sense pressure remotely.

Fig. 10 is a flow diagram of one embodiment of a method of reducing an influence of birefringence in a PM optical fiber on a pressure measurement obtained therethrough.

## DETAILED DESCRIPTION

It is realized herein that a need exists for a better remote pressure sensor. More specifically, it is realized herein that a need exists for a remote pressure sensor that is also more robust and reliable but also can accommodate additional functions, such as ultrasonic imaging. Introduced herein are various embodiments of a remote pressure sensor and method of operating the same

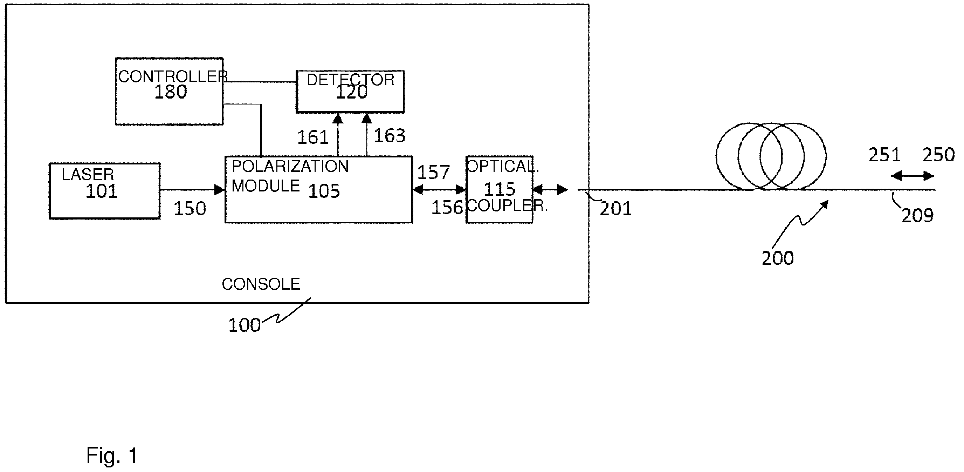

Fig. 1 is a schematic block diagram of one embodiment of a pressure sensor. The pressure sensor includes a long, flexible probe 200 and a console 100. At the probe's proximal end 201, close to the console 100, the probe 200 is attached to the console 100 mechanically and communicates with the console 100 optically and/or electrically. Such attachment can be achieved with a conventional or properly designed connector (not shown, but apparent to those skilled in the pertinent art). The console 100 comprises a laser 101, a polarization module 105, an optical coupler 115, a detector 120, and a controller 180. Linearly polarized Laser light 150 emitted from the laser 101 is incident on and passes through the polarization module 105, exiting as laser light 156 having an altered and/or modulated polarization state. The laser light 156 passes through the optical coupler 115 and is coupled into an optical fiber (not shown) embedded in or on the probe 200. The laser light 156 propagates inside the probe 200 towards the probe's distal end 209, becoming laser light 250 at the tip (shown but not referenced in Fig. 1) of the distal end 209. The laser light 250 is then reflected by a reflective coating (not shown in Fig.

1) at the tip, becoming laser light 251, which propagates back towards the proximal end 201 of the probe 200. The back-propagating light exits the probe 200 and passes through the coupler 115 again, exiting the coupler 115 as laser light 157. As the laser light 157 back-propagates through the polarization module 105, it is split into two laser light beams 161, 163, which are directed towards the detector 120. The detector 120 has two input ports (unreferenced), each receiving one of the two laser light beams 161, 163, and is configured to generate an electrical output signal that is proportional to the difference between the optical powers of the laser light beams 161, 163. The controller 180 receives the electrical output signal from the detector 120 and also communicates electrically with the rest of the console, including the polarization module 105. The controller 180 is configured to process the electrical output signal from the detector 120 to obtain the value of the pressure at the distal end 209 of the probe 200.

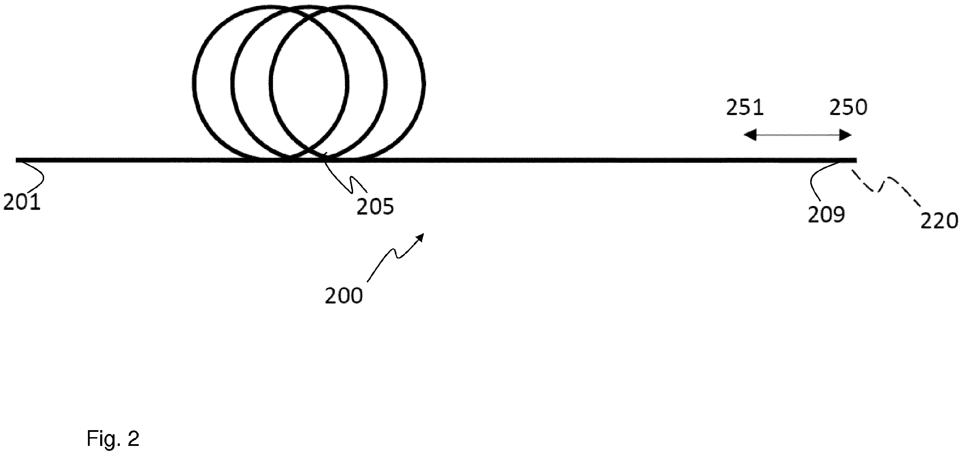

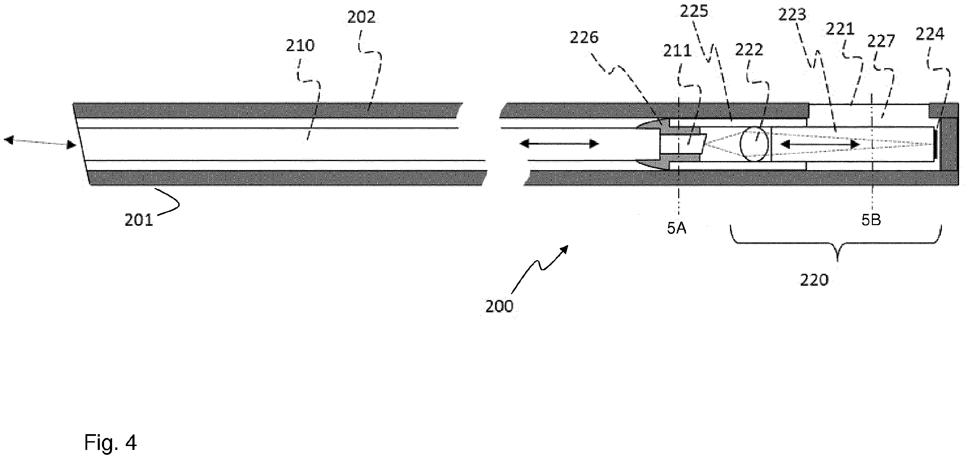

Fig. 2 is a diagram of certain parts of one embodiment of the probe 200. The probe 200 includes a long, flexible main body 205, the proximal end 201, and the distal end 209. A polarization maintaining ("PM") optical fiber (not shown in Fig. 2, but shown in Fig. 4) is embedded inside the probe 200 and is properly terminated at the proximal end 201 and distal end 209 as described below in conjunction with Fig. 4. A sensor 220 exists at the distal tip 209 of the probe 200. Laser light from console 100 is coupled into the PM optical fiber at the proximal end 201. The laser light propagates in the PM optical fiber in both the forward (towards distal end 209) and backward (towards proximal end 201) directions, because the forward-propagating laser light 250 is reflected by a reflective coatingin the sensor 220 at the distal tip 209 of the probe 200 and becomes the backward-propagating light 251.

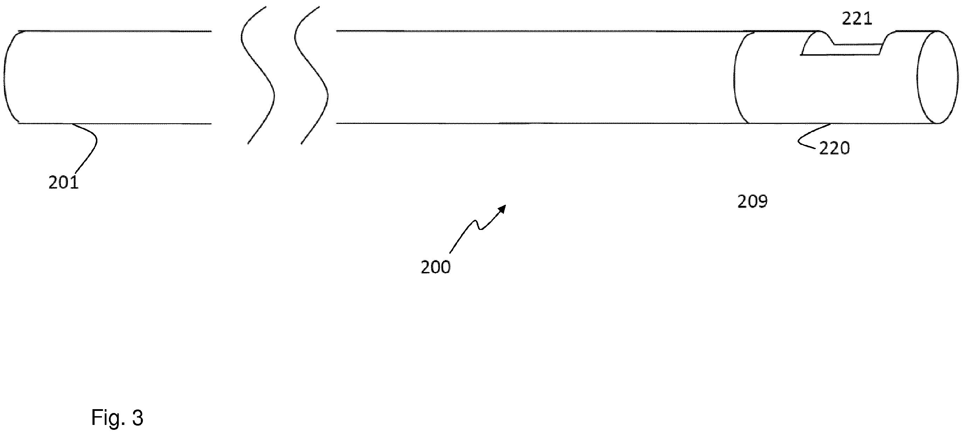

Fig. 3 illustrates the sensor 220 at the distal tip of the probe 200. The sensor 200 has a window 221 that provides a direct path for the internal components of the sensor 200 to couple mechanically with the outside environment. The pressure surrounding the sensor 200 stresses the internal components of the sensor 200 primarily through the window 221, creating a change in the optical anisotropy property of a material in the sensor 200. The change in optical anisotropy causes a change in the polarization state of the laser light propagating inside the material constituting the internal components. When the back-propagating light 251 re-enters the console (100 of Fig. 1) and reaches the detector (120 of Fig. 1), the detector generates an electrical output signal that substantially (within 10%) correlates with the pressure value at the distal tip of the probe. The nature of this mechanism will be described in greater detail below.

Fig. 4 is a diagram showing the details of one embodiment of the construction of the probe 200 of Fig. 2. The main body of the probe 200 comprises a PM optical fiber 210 embedded inside a flexible hypotube 202. At the proximal end of the probe, the PM optical fiber 210 is terminated with an angle polish. This is done to eliminate undesired reflections of laser light back into the console (100 of Fig. 1), and is a feature commonly implemented in conventional fiber optical connectors for telecommunications. Near the distal tip of the probe, the PM optical fiber 210 terminates inside a ferrule 225. As is often the case with fiber termination, the PM optical fiber 210 can be partially stripped of its protective jacket to expose the glass core 211 inside the ferrule 225. The PM optical fiber 211 is optically polished at a suitable angle, again to avoid undesired back-reflections, and it is bonded to the ferrule by epoxy 226 in the illustrated embodiment. The forward-propagating light exiting the fiber 211 enters the sensor 220 comprising a microlens 222, a photoelastic material 223 bonded to the ferrule 225 and encased in a proper fillant 227, such as a silicone elastomer, an opening 221, and a reflective coating 224 on the distal tip of the photoelastic material 223. In various embodiments, the photoelastic material 223 may be selected from various types of glasses, such as fused silica, or polymer plastics, such as polycarbonate. The forward propagating light is reflected by the coating 224 to back-propagate through the same components in the sensor and into the fiber 211.

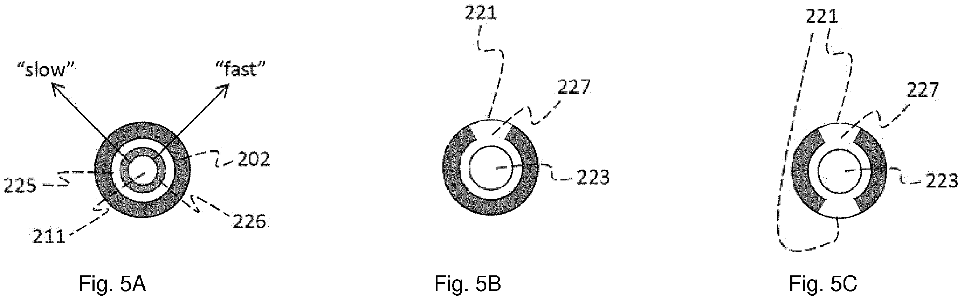

Figs. 5A and 5B are cross-sectional views of the sensor of Fig. 4, taken along respective lines 5A and 5B thereof. The view of Fig. 5A shows the fiber 211 bonded by epoxy 226 to a ferrule 225 housed inside a flexible hypotube 202. Polarization-maintaining optical fibers, such as the fiber 211, are usually birefringent single-mode fibers whose "fast" and "slow" axes are often called the "principal axes" of the fiber. The view of Fig. 5B shows the photoelastic material 223 encapsulated in fillant 227, inside a hypotube 202 that has an opening 221 pre-cut on it. Note that the opening 221 is substantially ($\pm 5°$) along the direction that is 45° from the principal axes of the PM optical fiber 211. Fig. 5C is a cross-sectional view of the sensor of Fig. 4, taken along lines 5B and showing an alternative embodiment of the view of Fig. 5B. As Fig. 5C shows, the opening 221 can optionally be cut symmetrically on both sides of the hypotube 202.

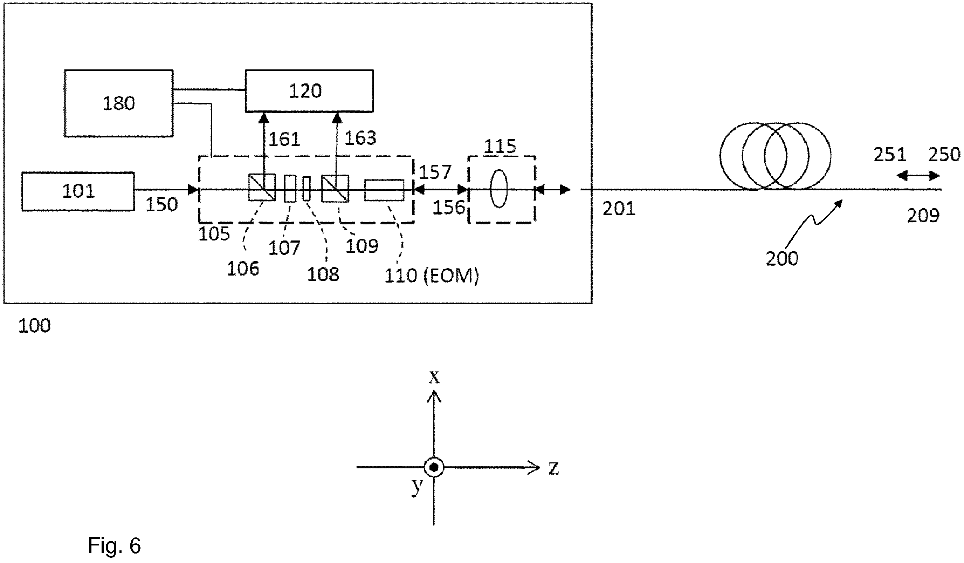

Fig. 6 is a diagram of one embodiment of the polarization module 105 of Fig. 1. This embodiment of Fig. 6 includes polarizing beam splitters ("PBSs") 106, 109, a half-wave plate 107, a Faraday rotator 108, and an electro-optic modulator ("EOM") 110. In embodiment, the EOM 110 is a Pockels cell, including an electro-optic crystal such as LiTaO3 with the proper cut and polish, and with plated electrodes. For clarity of discussion, Fig. 6 also shows a Cartesian coordinate system, in which the z-axis is the direction of forward-propagating light and the x- and y-axes are in the plane perpendicular to the z-axis. The incident light 150 is polarized along the x-axis and is substantially (at least 90%) transmitted through the PBS 106. The half-wave plate 107 rotates the light polarization to an angle of 45° in the x-y plane. However, as light propagates forward through the Faraday rotator 108, the polarization is rotated back to be along the x-axis again, enabling it be substantially transmitted through the PBS 109. The optical axis of the electro-optic modulator 110 is at about 45° in the x-y plane, so the polarization state of the light 156 after passing through the EOM 110 is strongly dependent on the voltage applied on the EOM 110. After being focused by a coupling lens 115, laser light enters the PM optical fiber (not shown) in the probe 200. Note that in Fig. 6, at the probe's proximal end 201, the principal axis of the PM optical fiber is assumed to be parallel to the optical axis of the EOM 110, at 45° in the x-y plane. If the proximal end 201 of the probe 200 needs to be oriented independently, the principal axis of the PM optical fiber is not guaranteed to be parallel to the optical axis of the EOM 110. In this case, a half-wave plate (not shown in Fig. 6) may be added at a location between the EOM 110 and the proximal end 201 of the probe 200 effectively to align the principal axis of the PM optical fiber to the optical axis of the EOM 110.

Because of the optical axis alignment noted above, the forward propagating light in the PM optical fiber experiences a birefringence from the fiber that directly adds to the birefringence experienced from the EOM 110. However, as light reaches the photoelastic material 223 at the distal tip 209 of the probe 200, the birefringence experienced there cannot be added directly to the birefringences experienced from either the PM optical fiber or the EOM 110. The photoelastic material 223 gains a birefringence that is approximately proportional to the pressure-induced stress it experiences, primarily along the direction of the stress. Referring back to Fig. 5, the sensor's opening 221 is at an angle of about 45° from the principal axis of the PM optical fiber 211. Thus the stress that the outside pressure induces on the photoelastic material 223 is primarily along the same direction, and so is the pressure-induced birefringence. Because the pressure-induced birefringence in the photoelastic material 223 is along a direction approximately 45° from the principal axis of the PM optical fiber 211, it cannot be added directly to the birefringences experienced from either the PM optical fiber 211 or the EOM 110.

As noted before, light reflected by the reflective coating 224 traverses the photoelastic material 223 again, propagates back inside the PM optical fiber, towards the proximal end of the probe, and re-enters the console 100. As stated above, the polarization module 105 splits the back-propagating light into two light beams 161, 163. The balanced detector 120 receives the two light beams at its two input ports respectively, and produces an electrical output voltage that is proportional to the difference in the optical power of the two input light beams.

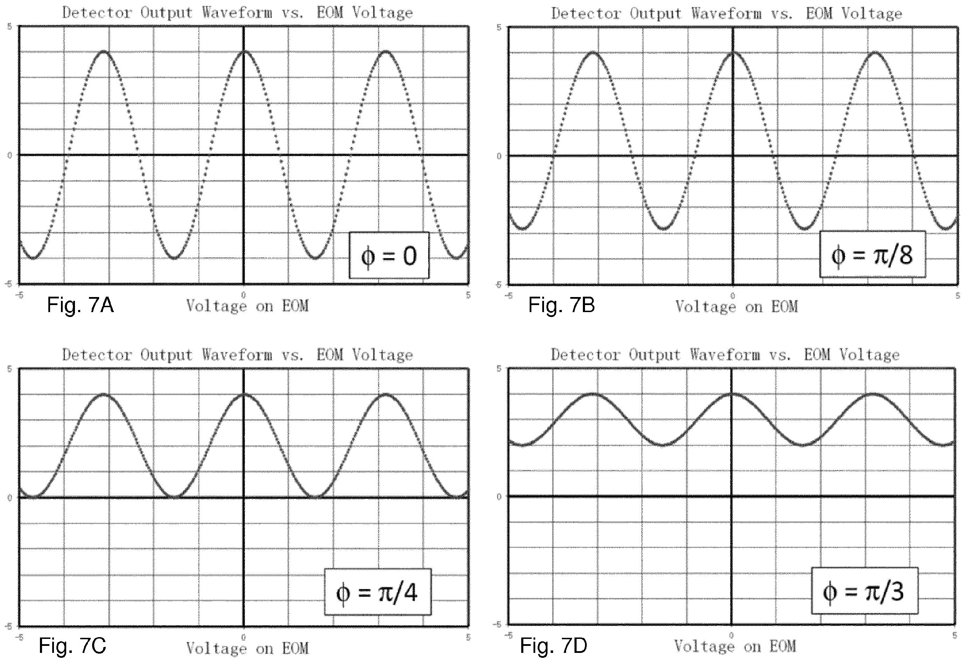

Jones Calculus may be employed to determine how the detector output signal changes as a function of the amount of birefringence experienced at the photoelastic material 223. Figs. 7A-7D illustrate four example simulated results. Each of Figs. 7A-7D shows a waveform representing the output voltage of the detector 120 as an applied voltage on the EOM 110 is swept. Thus, each of Figs. 7A-7D corresponds to the detector output waveforms for four different pressures-induced birefringences present at the photoelastic material 223. The birefringent phase shift φ is defined as:

$$\phi = 2\pi * \Delta n * L / \lambda,$$

where φ is the (single-pass) birefringent phase shift of the photoelastic material, and Δn is the amount of pressure-induced birefringence, measured as a change in refractive index. L is the length of the photoelastic material, and λ is the optical wavelength of laser light in vacuum. As is seen in of Figs. 7A-7D, when the photoelastic material 223 induces no birefringence (φ=0), the waveform is a perfect sinusoid. However, as φ increases, the bottom of the waveform rises, and the waveform becomes evermore shallow. In the most extreme case, when φ = π/2, the waveform would rise to the top and become a straight line (not shown).

Therefore, a controller 180 in the console can process the detector waveform and derive information about the birefringent phase shift φ, and, in turn, determine the pressure-induced birefringence Δn. Since for a given photoelastic material, the relationship between pressure and Δn is known or can be measured beforehand, the pressure at the distal tip of the probe can be determined from the birefringence measurement above.

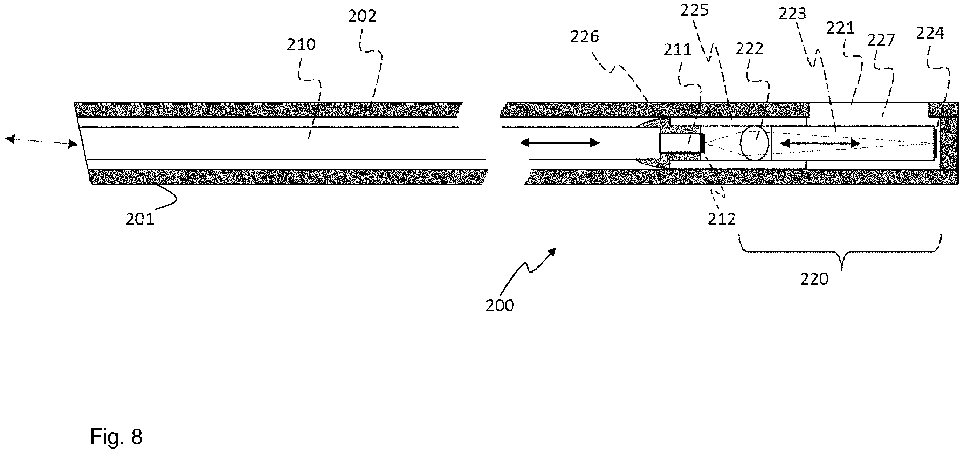

Sometimes it is desirable to be able to measure the birefringence of the PM optical fiber in the probe without substantial interference from the sensor in the probe. The embodiments described herein can potentially be calibrated in real-time (during use), to account for and compensate for any deviations of the PM optical fiber from ideal behavior. Fig. 8 is a diagram of one embodiment of a pressure probe that can be calibrated using a second wavelength. The difference between this embodiment and the one shown in Fig. 4 is the addition of a wavelength-selective coating 212 at the tip of the terminated PM optical fiber 211. In addition, the polishing angle of the fiber tip 211 can be substantially (±5°) closer to normal (a perpendicular polish). The wavelength-selective coating 212 acts as an anti-reflection coating for the first wavelength, substantially (at least 90%) passing all light with that wavelength. The wavelength-selective coating 212 also acts like a high reflectivity coating for the second wavelength, which in the illustrated embodiment, is close to the first wavelength.

Since light of second wavelength is substantially reflected (at least 90%) by the wavelength-selective coating 212, the photoelastic material 223 does not significantly affect its birefringence. Therefore, if the console (100 of Fig. 1) has the ability to launch light of the second wavelength into the PM optical fiber, the console can perform an independent measurement of the birefringence of the PM optical fiber. This measurement result effectively serves as real-time "background data." When the console launches light of first wavelength into the PM optical fiber to measure pressure at the distal tip, the background measurement above can provide the means to compensate for imperfections in the PM optical fiber, thus achieving a more accurate pressure measurement.

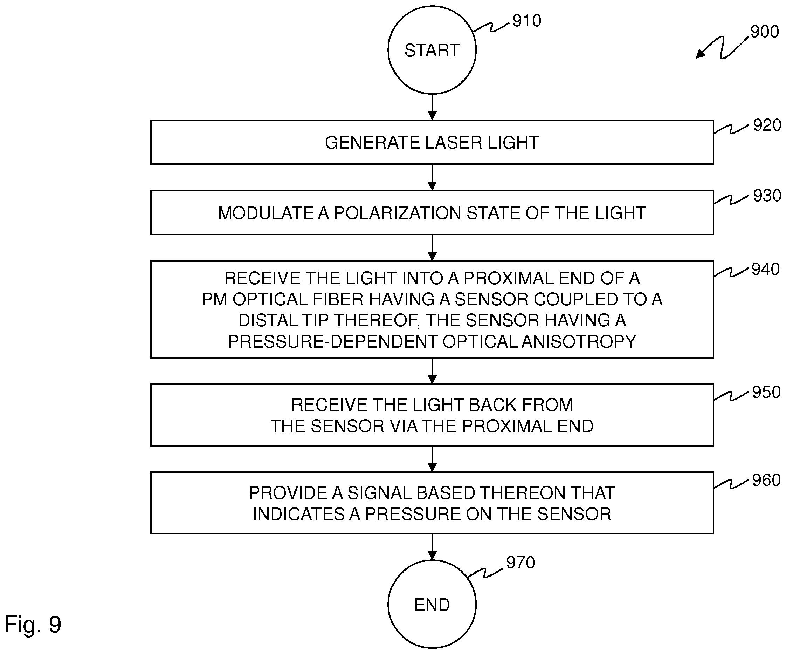

Fig. 9 is a flow diagram of one embodiment of a method 900 of operating a remote pressure sensor to sense pressure remotely. The method 900 begins in a start step 910. In a step 920, laser light is generated. In a step 930, a polarization of the light is modulated. In a step 940, the light is received into a proximal end of a PM optical fiber having a sensor coupled to a distal tip thereof, the sensor having a pressure-dependent optical anisotropy. In a step 950, the light is received back from the sensor via the proximal end. In a step 960, a signal based thereon is provided, the signal indicating a pressure on the sensor. The method 900 ends in an end step 970.

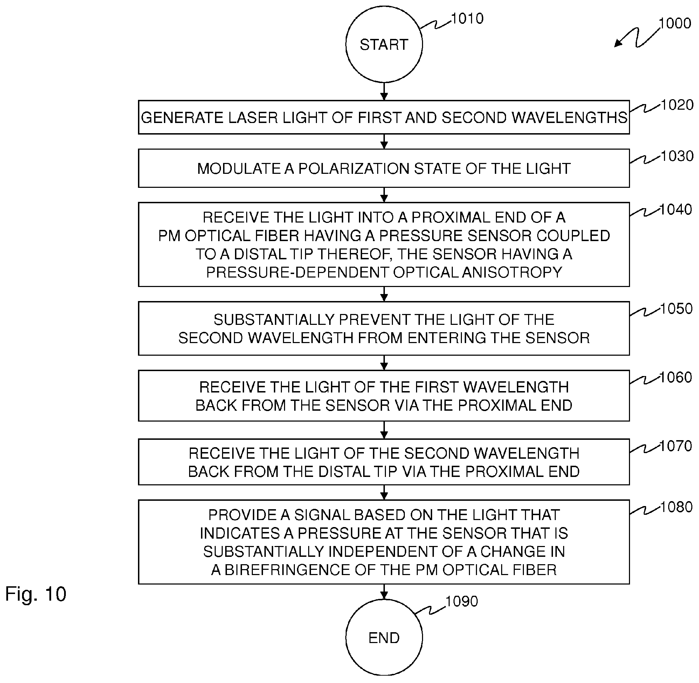

Fig. 10 is a flow diagram of one embodiment of a method 1000 of reducing an influence of birefringence in a PM optical fiber on a pressure measurement obtained therethrough. The method 1000 begins in a start step 1010. In a step 1020, laser light of first and second wavelengths is generated. In a step 1030, the polarization state of the light is modulated. In a step 1040, the light is received into a proximal end of a PM optical fiber having an optical sensor coupled to a distal tip thereof, the sensor having a pressure-dependent optical isotropy. In a step 1050, the light of the second wavelength is substantially (at least 90%) prevented from entering the sensor. In a step 1060, the light of the first wavelength is received back from the sensor via the proximal end. In a step 1070, the light of the second wavelength is received back from the distal tip via the proximal end. In a step 1080, a signal based on the light is provided that indicates the pressure at the sensor that is substantially (at least 90%) independent of change in birefringence of the the PM optical fiber. The method 1000 ends in an end step 1090.

Those skilled in the art to which this application relates will appreciate that other and further additions, deletions, substitutions and modifications may be made to the described embodiments.

**Claims**

1. A pressure sensor, comprising:

   a source of laser light;
   a polarization module coupled to said source and configured to modulate a polarization state of said light;
   a polarization maintaining (PM) optical fiber configured to receive said light into a proximal end thereof and having a sensor coupled to a distal tip of said PM optical fiber and having a pressure-dependent optical anisotropy; and

a detector configured to receive said light back from said sensor via said proximal end and provide a signal based thereon that indicates a pressure on said sensor.

2. The pressure sensor as recited in Claim 1 wherein said sensor is oriented with respect to said distal tip such that said optical anisotropy lies along an axis that is 45°±5° with respect to a principal axis of said PM optical fiber.

3. The pressure sensor as recited in Claim 1 or 2 wherein said sensor includes a photoelastic material having a reflective coating configured to reflect at least some of said light incident thereon.

4. The pressure sensor as recited in Claim 3 wherein said reflective coating is wavelength-dependent.

5. The pressure sensor as recited in any one or more of Claims 1 to 4 wherein said source and said detector are located in a console coupled to said proximal end.

6. The pressure sensor as recited in Claim 5 wherein said signal is proportional to a difference between optical powers of light received from said polarization module.

7. A method of reducing an influence of birefringence in a polarization maintaining (PM) optical fiber on a pressure measurement obtained therethrough, comprising:

generating laser light of first and second wavelengths;

modulating a polarization state of said light;

receiving said light into a proximal end of said PM optical fiber having an optical sensor coupled to a distal tip thereof, said sensor having a pressure-dependent optical anisotropy;

substantially preventing said light of said second wavelength from entering said sensor;

receiving said light of said first wavelength back from said sensor via said proximal end;

receiving said light of said second wavelength back from said distal tip via said proximal end; and

providing a signal based on said light that indicates a pressure at said sensor that is substantially independent of a change in a birefringence of said PM optical fiber.

8. The method as recited in Claim 7 further comprising:

causing said light to pass through a polarization module; and

compensating for said change by adjusting a bias in a said polarization module.

9. The method as recited in Claim 7 or 8 wherein said sensor is oriented with respect to said distal tip such that said optical anisotropy lies along an axis that is 45°±5° with respect to a principal axis of said PM optical fiber.

10. The method as recited in any one or more of Claims 7 to 9 wherein said sensor includes a photoelastic material having a reflective coating, said method further comprising reflecting at least some of said light incident on said coating.

LASER 101

CONTROLLER 180

DETECTOR 120

POLARIZATION MODULE 105

OPTICAL COUPLER 115

CONSOLE 100

150

161

163

157

156

201

200

209

250

251

Fig. 1

Fig. 2

EP 3 070 449 A1

Fig. 3

Fig. 4

Fig. 5C

Fig. 5B

Fig. 5A

Fig. 6

EP 3 070 449 A1

Detector Output Waveform vs. EOM Voltage

$\phi = 0$

Fig. 7A          Voltage on EOM

Detector Output Waveform vs. EOM Voltage

$\phi = \pi/8$

Fig. 7B          Voltage on EOM

Detector Output Waveform vs. EOM Voltage

$\phi = \pi/4$

Fig. 7C          Voltage on EOM

Detector Output Waveform vs. EOM Voltage

$\phi = \pi/3$

Fig. 7D          Voltage on EOM

Fig. 8

Fig. 9

Fig. 10

EP 3 070 449 A1

Europäisches
Patentamt

European
Patent Office

Office européen
des brevets

## EUROPEAN SEARCH REPORT

Application Number

EP 16 16 0451

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/199773 A1 (ZHOU GAN [US]) 12 August 2010 (2010-08-12) * abstract * * paragraphs [0029], [0033] * * figures 1,2 * | 1-10 | INV. G01L11/02 G01H9/00 G01L1/24 |
| A | US 5 987 995 A (SAWATARI TAKEO [US] ET AL) 23 November 1999 (1999-11-23) * abstract; figures 1-3, 6 * | 1 | |

TECHNICAL FIELDS
SEARCHED (IPC)

G01L
G01H

The present search report has been drawn up for all claims

| Place of search | Date of completion of the search | Examiner |
|---|---|---|
| The Hague | 2 August 2016 | Kister, Clemens |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 16 0451

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2016

| Patent document cited in search report | | Publication date | Patent family member(s) | Publication date |
|---|---|---|---|---|
| US 2010199773 | A1 | 12-08-2010 | NONE | |
| US 5987995 | A | 23-11-1999 | NONE | |

EPO FORM P0459

For more details about this annex : see Official Journal of the European Patent Office, No. 12/82